# EUROPEAN PATENT APPLICATION

(11) **EP 1 498 475 A1**
(43) Date of publication of application: **19.01.2005**
(21) Application number: 03291790.8
(22) Date of filing: 18.07.2003
(51) Int. Cl.: C12M 3/00, C12M 1/40, C12N 9/00, C12N 5/04

(54) **Continuous plant cell bioreactor and method for continuous plant cell culture**

(71) Applicant: MERISTEM THERAPEUTICS S.A., 63100 Clermont-Ferrand (FR)
(72) Inventor: Maillard, Patrick, 63400 Chamalières (FR); Wallis, Gregg, 63130 Royat (FR); Langlais, Audrey, 63000 Clermont Ferrand (FR)
(74) Representative: Thurgood, Alexander John

(57) **Abstract**

The present invention concerns a continuous plant cell bioreactor and a method for continuous plant cell culture. The continuous plant cell bioreactor of the invention comprises a fermentation chamber, an oxygenating inlet enabling oxygenating fluid to enter the chamber, a cell media inlet enabling cell media to enter the chamber, and a plant cell supernatant outlet enabling cell supernatant to exit the chamber, wherein the cell media inlet and the supernatant outlet are located substantially one opposite the other. The method and apparatus can be used to continuously produce recombinant therapeutic proteins from plant cell culture.

## Description

The present invention relates to bioreactors and in particular a plant cell bioreactor. The invention will be more particularly described and explained with respect to an exemplified continuous plant cell bioreactor and a method for culturing plant cells continuously.

Higher plants are valuable sources for a variety of biochemicals (peptides, secondary metabolites) which are used as drugs, pesticides, insecticides, flavors, fragrances and other fine chemicals. With the development of genetic engineering, production of transgenic plants expressing high value proteins such as recombinant therapeutic proteins has been made possible. However, characteristics of recombinant proteins produced in plants often need to be rapidly evaluated. To this end, feasibility tests have been developed, for example, transient expression systems, that can give enough protein for characterization in only a few days. For an intermediate scale production of secreted protein, plant cell suspension represents an interesting model. Culture medium generally contains a comparatively low level of proteins with respect to regenerated plant tissues or cells, and enrichment of the recombinant protein is often necessary. Many recombinant proteins have been produced in suspensions of cell cultures, cultivated in flasks on rotative shakers. However production of a few milligrammes of protein requires the scale up of the culture. Many bioreactor systems have been designed for the mass culture of plant cells and a number of plants species have been used, including *Arabidopsis, Catharanthus, Taxus, Thalictrum* and *Nicotiana* even at an industrial scale. In general, the plant cell culture uses a fermenter with applied standard modes like batch, fed-batch and continuous. However, current continuous mode cell suspension culture has shown its limits in that the duration of the culture has only been a few days at most because plant cells are very sensitive to mechanical and hydrodynamic stresses. This has made industrial scale continuous plant cell suspension culture uneconomical from an industrial point of view.

It is therefore an object of the present invention to provide a continuous culture system that is capable of functioning on an industrial scale. In this way, the protein purification process could also then be continuously performed as long as the cells remained alive. This would moreover reduce cost when compared to the known batch culture systems, due to lower reactor immobilization times.

Accordingly, one of the objects of the present invention is a continuous plant cell bioreactor comprising :
- a fermentation chamber;
- an oxygenating inlet enabling oxygenating fluid to enter the chamber;
- a cell media inlet enabling cell media to enter the chamber;
- and a plant cell supernatant outlet enabling cell supernatant to exit the chamber;
wherein the cell media inlet and the supernatant outlet are located substantially one opposite the other.

The applicant's have in effect serendipitously discovered that in order for continuous cell culture to be viable in the bioreactor over and beyond the known periods of about four days, it is necessary to modify the arrangement of the bioreactor as outlined above. No-one thought this important previously, but the present applicants have discovered that if the cell media inlet and the supernatant outlet are located too close to each other or are one and the same, then rapid plant cell death will entail after a period of about four days of continous culture.

There are currently two bioreactor systems used for cell suspension culture : stirred-tank reactors and airlift reactors. For example, stirred-tank bioreactors have been used for large-scale ginseng production using *Panax ginseng,* while airlift bioreactors have been used for alkaloid production with *Catharanthus roseus* or *Thalictrum rugosum.* The two systems have also been used with success for *Nicotiana tabacum* plant cell culture, but it is generally admitted that the airlift bioreactor has the advantage of having a low shear, low energy requirement and a simple design. Accordingly, in a preferred embodiment of the present invention, the reactor is a stirred tank bioreactor or an airlift bioreactor, and more preferably is an airlift bioreactor.

In an airlift bioreactor, three basic systems for circulating biomass are known :
- an external-loop airlift reactor, where risers and downcomers are separate tubes connected by short horizontal sections at the top and bottom;
- an internal-loop airlift reactor, in which the riser and the downcomer are separated by an internal baffle or draft tube, and;
- a bubble column reactor, which has the advantage of being easily sterilized using an autoclave, and for which maintenance is minimal.

One of the problems of such a bubble column system is that little is known about fluid flow within it, and mixing is non uniform. For example, Kato *et al.* (1976) have observed a reduced growth rate of *N. tabacum* cells cultivated in a bubble column bioreactor because of insufficient mixing in the reactor.

The present applicants have sought also to overcome the disadvantages of using such known bubble column systems, and have managed to provide a method and apparatus that enables long-term, continuous, and industrially scalable plant cell suspension culture for producing, for example, recombinant proteins, comparable to what can be achieved with mammalian or yeast cell culture.

In one preferred embodiment of the invention, the reactor is a stirred tank bioreactor or an airlift bioreactor, and preferably is an airlift bioreactor. In this case, it is most preferred when the airlift bioreactor is chosen from the group consisting of an external-loop airlift reactor, an internal-loop airlift reactor, and a bubble column reactor, and even more preferably the bioreactor is a bubble column airlift bioreactor.

The bioreactor of the present invention comprises a fermentation or reactor chamber, that will receive plant cell suspension and growth media in the form of a cell medium. The terms "fermentation chamber" and "reactor chamber" will be used indifferently in the specification and claims to mean the same thing. The chamber can have any suitable shape, such as spherical, cuboid, cylindrical, but preferably is substantially elongate in shape, preferably substantially rectangularly cubic, and even more preferably, substantially cylindrical. Such a design allows for several such chambers to be set up in a minimal amount of space, and these chambers can all be controlled from one command or control unit. The chamber is generally made of glass, but could be made of any suitable material that is resistant to the operating conditions being used, for example, high impact or reinforced plastics, stainless or galvanized steel, or aluminium, titanium, alloys thereof and the like. The size of the fermentation chamber is variable, and can be scaled up for industrial production. For example, the chamber can have a volume ranging from ¼ liter to 15 liters or even more. However, for reasons linked to easy and convenient handling of the material and pressure resistance, preferred volumes are however comprised between 2.5 liters and 10 liters. Where larger chambers are involved, say for example, chambers of about 10 liters in volume or more, one can provide expansion rings, that are well known to the skilled person, to avoid deformation of the chamber. A suitable example of a chamber that can be used in the present invention is a an approximately 2.5 liter glass chamber about 55 cm in total height, with a useful height of about 40 cm and a diameter of about 10 cm. By useful height, it is to be understood the height of the chamber that can actually be used to accommodate cell media and enable practical cell culture. In one particularly preferred embodiment, the bottom of the fermentation chamber has substantially sloping walls that converge towards the oxygenating fluid inlet. Indeed, the applicant has discovered that such sloping walls bring an added advantage to the system in that they force cell material that falls under the effect of gravity to be pushed down towards the oxygenating fluid inlet, rather than accumulating in corners of the chamber, and thereby obviates the need for a stirring or agitation mechanism that are usually found in cell suspension bioreactors. These walls therefore improve circulation of the cell suspension within the chamber and increase the viability and hence the productivity of the bioreactor, enabling it to run for longer before cell death occurs.

It has been stated previously that the bioreactor has a cell media inlet for enabling cell media to enter the chamber. A separate inlet, called an inoculating suspension inlet can also be provided that enables introduction of an inoculating or 'mother' suspension to be introduced into the chamber. Preferably, however, the cell media inlet also serves as the inoculating suspension inlet, that is to say, both the inoculating plant cell suspension and the corresponding growth media and stimulating nutrients are introduced into the chamber via the same inlet.

The applicant has ascertained that it is particularly advantageous, and therefore preferred to locate the cell media inlet, the optional inoculating suspension inlet and the plant cell supernatant outlet substantially at the top of the chamber. By 'substantially', it is meant that these inlets are located at the upper end of the chamber. As mentioned previously, one of the important discoveries of the applicants that led to the present invention was the fact that the cell media inlet be substantially opposite the cell supernatant outlet. Indeed, should this not be the case, the distribution of the cell suspension within the chamber will not be optimal and will consequently lead to accelerated cell death, thereby shortening the operating time and hence reducing the productivity of the system to the extent that the bioreactor would function more like a batch culture system rather than a continuous one.

According to another preferred embodiment of the present invention, the oxygenating fluid inlet is located substantially at the bottom of the chamber. In this way, the oxygenating fluid, when introduced into the bottom of the chamber will rise substantially vertically along the longitudinal axis of the chamber and substantially parallel to the longitudinal walls of the chamber. Upon rising, the oxygenating fluid also lift cells from the bottom of the chamber that have fallen back down under the effects of gravity and that have been brought to the oxygenating inlet by the sloping walls of the chamber. Naturally, it is preferred in such a case to use an oxygenating fluid that has a density that is lower than the density of the cell culture in the chamber, in order that the cells and the extracellular fluid are lifted up by the movement of the rising oxygenating fluid. Alternatively, the oxygenating fluid can be forced up the chamber by introducing it under pressure into the bottom of the chamber. Another possibility is to use an oxygenating fluid of lower density and also introduce said fluid under pressure into the bottom of the chamber. In a preferred embodiment, the oxygenating fluid is air or substantially pure gaseous oxygen, but air is most preferred since it represents a good compromise between oxygenating capacity and oxidative destruction capacity. Preferably, the oxygenating inlet comprises a filter, to avoid any introduction of foreign bodies that might lead to accelerated cell death, more preferably, the oxygenating inlet comprises a sintered glass filter.

The applicant has also determined that the filter needs to be of a certain porosity, in order that the bubbles of oxygenating fluid introduced into the chamber are not so big that they cause excessive cell degradation through their oxidative capacity, but that they are sufficiently large to be able to carry the cells up the chamber and still provide oxygen to the cells. The oxygenating inlet therefore preferably comprises a filter having a pore diameter comprised between about 17 micrometers and about 100 micrometers. The size of the filter is also linked to the flow rate of oxygenating fluid introduced into the chamber. In particular, the applicants have discovered that the best performance can be achieved when the flow rate is such that sedimentation of the cells in suspension is not too important, but also such that the cells are not degraded by the oxidative effect of the oxygenating fluid. In this case, the applicant has determined a particularly useful flow rate to be comprised from about 0.1 vvm to about 1 vvm, and preferably from about 0.1 vvm to about 0.7 vvm. Indeed, a flow rate greater than 1 vvm has been found to cause too extensive oxidative damage and shorten the lifespan of the cells, and hence the operability of the reactor. Optionally, wherein at least one filter is further provided between the oxygenating fluid source and the oxygenating fluid inlet.

Another parameter that is regulated during cell culture is the reactor temperature. Preferably, the operational reactor temperature is comprised between about 23°C and about 27°C. This temperature range has been determined as the optimal range of operation of the bioreactor according to the present invention. Accordingly, the bioreactor also comprises a chamber temperature regulation system. Such a system can be, for example, and preferably, a water-cooled chamber temperature regulator system disposed around an exterior peripheral wall of the chamber. An example of such a system would be a second chamber substantially enclosing the fermentation chamber and through which or in which cooling water is circulated. Of course, the skilled person will realize that other forms of temperature regulation systems known to him could be appropriately used to attain the same effect, for example without the use of water as temperature regulating fluid, and such temperature regulation systems can be in direct or indirect contact with the exterior peripheral wall of the chamber. In the present invention, the cooled water preferably enters the regulator system near the bottom of the chamber. In a variant, the cooled water can also enter the regulator system near the top of the chamber. Most preferably, the water- cooled regulator system is coiled around an exterior peripheral wall of the chamber.

As has been mentioned previously, the bioreactor according to the invention has a supernatant outlet that enables supernatant, containing cell debris and extracellular liquid in which the expressed substances are contained, to exit the chamber. Preferably, this outlet is located below an upper level of a cell culture in the chamber, so that the excess liquid, or supernatant, runs off spontaneously with the movement generated by the movement of cells within the chamber.

In order to supply a constant stream of cell media, the bioreactor can also have at least one separate cell media tank connected to the cell media inlet, and preferably comprises two cell media tanks disposed in series, between which at least one filter is located. This ensures that the cell media entering the chamber is essentially sterile and avoids contamination of the cells by bacteria, or yeasts that could be fatal to the cell culture, or interfere with the production of the polypeptides or fragments that it is intended to recover and purify from the cell culture. Advantageously, the cell media also contains growth media comprising basic nutrients required for cell viability. In addition, the cell media can also preferably comprise an antioxidant in an amount comprised between about 2 to about 4 mg/litre. More preferably, the antioxidant is ascorbic acid. The cell media can advantageously be stored in a lightproof container, or a container shielded from the light to reduce degradation of any light sensitive antioxidant that may be present in the cell media.

In addition to the foregoing elements of the bioreactor, another preferred embodiment involves providing at least one harvesting tank, connected to the plant cell supernatant outlet. In this way, the supernatant liquid and cell debris is recovered into a separate tank, and from there can be stored or sent for further processing, for example to purify a recombinant therapeutic protein that has been expressed in the cell culture and has been secreted to the extracellular liquid. One preferred way of separating this mixture of cell debris and supernatant liquid is by passing the mixture through a tangential filter unit, more preferably located between the cell supernatant outlet and the harvest tank. In this way, it is possible to recover just the extracellular liquid containing the expressed substance of interest.

The reactor chamber can also comprise an exhaust fluid outlet for evacuating exhaust fluids from the chamber. Preferably, such exhaust fluids are evacuated naturally from the chamber, but the skilled person could of course provide for a forced extraction system, for example a pump system. Optionally the exhaust outlet is connected to a filter system to cleanse the exhaust fluids before they escape into the atmosphere.

As has been discussed previously, the invention concerns a continuous plant cell bioreactor and method therefor. Consequently, the cell media contained in the cell tanks is a suspension of plant cells, and preferably embryonic undifferentiated plant cells. In a more preferred embodiment, the plant cells used are transgenic plant cells expressing a recombinant polypeptide or peptide fragment. The plant cells that can be used are preferably cells from monocotyledonous or dicotyledonous plants, and even more preferably dicotyledonous plants from species such as *Arabidopsis,* duckweed, tobacco, carrot, potato, lettuce, beetroot, cabbage, rape, canola and the like. As mentioned, however, monocotyledonous plant cells can also be used, for example rice, maize, wheat, barley, sorghum, oats, corn and the like.

Even more preferably, the recombinant polypeptide or peptide fragment expressed by the cell suspension is a therapeutic protein. Exemplary recombinant therapeutic proteins are most preferably selected from the group consisting of antibodies, antigens, antibody fragments, antigen fragments, cardiovascular proteins, digestive enzymes, cerebro-stimulatory or modulatory enzymes, pulmonary enzymes, anti-tumour proteins, tissular proteins, immunostimulatory or modulatory proteins, and blood or blood derived proteins, bone morphogenesis proteins, and growth hormones. Such proteins might be, for example, gastric or intestinal lipases, hemoglobin or hemoglobin derivatives, human serum albumin, immunoglobulins or their fragments, glycoproteins, such as Rabies virus glycoproteins, HIV coat proteins, lactoferrin, fibrinogen, mono- or bikunines, calmodulins, hepatocyte growth factors, granulocyte macrophage colony stimulating factors, blood factors such as factors VIII, IX, X, XI and the like.

Finally, the bioreactor can also comprise a control unit for regulating operation of the reactor and the various tanks, filters, pumps, temperature regulation systems that form part of it. This brings a level of comfort and security to the operator, since it lowers human error and enables an industrial scale up of the reactor.

Yet another object of the present invention is a method of producing recombinant proteins through continuous culture of the cell suspension that is possible over a far longer period of time than has been previously possible. Accordingly, one object of the invention is a method for the continuous culture of a plant cell suspension, comprising :
- introducing a plant cell suspension into a fermentation chamber;
- introducing an oxygenating fluid into the fermentation chamber
whereby the oxygenating fluid is introduced in such a way as to define a circulatory path sufficient to maintain the viability of the plant cell suspension. Preferably, the chamber used in the method is substantially as described in the preceding part of this application.

In a preferred embodiment of the method of this invention, the cell media is introduced into the chamber via the cell media inlet substantially opposite to the cell supernatant outlet. Even more preferably, the plant cell suspension has a dry weight comprised between about 0.2 and about 6 g/L. Additionally, the plant cell suspension has a packed cell volume (PCV) comrpised between about 1 and 50%. Packed cell volume and dry cell weight are determined as follows :
A 100 mL plant cell culture sample is centrifuged with a Jouan C 4.11 centrifuge for 5 min at 1000 rpm (available from Jouan, France. The pellet is filtered through a 0.45 µm Wheaton glass sinter, and washed with distilled water. The water is removed by draining under vacuum until no further drops of water appeared. The biomass is placed on Whatman N°1 filter paper and the fresh weight measured on a pre-weighed Petri dish. The pellet is dried for 12 h at 80°C and the dry weight recorded. The percent of dry weight is the ratio (fresh weight/dry weight) x 100.

The packed cell volume (PCV) is determined by centrifuging 5 mL culture suspension in a 15 mL graduated conical centrifuge tube at 1000 rpm for 5 min. The fraction of the whole volume occupied by the cells determined the PCV.

Preferably, the oxygenating fluid is introduced at the bottom of the fermentation chamber.

Even more preferably, the fermentation or reactor chamber is maintained at an operating temperature comprised between about 23 to about 27°C.

Most preferably, the oxygenating fluid is air, and the oxygenating fluid is introduced at a flow rate comprised between about 0.1 vvm to about 1 vvm, and even more preferably from about 0.1 vvm to about 0.7 vvm. Most preferably, the oxygenating fluid is introduced in such a way as to produce bubbles having an average diameter at the point of introduction comprised between about 17 micrometers and 100 micrometers. Optionally, the oxygenating fluid is passed through a sintered glass filter just prior to introduction into the chamber via the oxygenating fluid inlet. In order to obtain still even better oxygenation of the cell suspension, it is preferred to additionally and periodically introduce oxygenating fluid in bursts at a greater flow rate than the operational flow rate.

In another preferred embodiment, the cells in suspension are made to fall onto the lower walls of the chamber that slope towards the oxygenating inlet.

In yet another preferred embodiment of the present method, the cell media contained in the cell tanks is a suspension of embryonic undifferentiated plant cells, and more preferably is a suspension of transgenic plant cells expressing a recombinant polypeptide or peptide fragment. Advantageously, and in a preferred manner, the recombinant polypeptide or peptide fragment expressed by the cell suspension is a therapeutic protein. Most preferably, the therapeutic protein is selected from the group consisting of antibodies, antigens, antibody fragments, antigen fragments, cardiovascular proteins, digestive enzymes, cerebro-stimulatory or modulatory enzymes, pulmonary enzymes, anti-tumour proteins, tissular proteins, immunostimulatory or modulatory proteins, and blood or blood derived proteins, bone morphogenesis proteins, and growth hormones.

In a more preferred manner, the method of the invention involves collecting the cell supernatant by overflow of the cell suspension in the chamber into the cell supernatant outlet. In another preferred embodiment of the method according to the present invention, the cell supernatant containing an expressed protein of interest is separated in a tangential filter to separate out cell debris and recover the supernatant.

Additionally, the exhaust fluids produced in the chamber can be exhausted via an exhaust outlet naturally.

The applicants have found, through the inventive apparatus and method according to the invention, that it was possible :
- to obtain excellent homogenization of the cell culture;
- to reduce cell adherence on the surface of the culture chamber;
- to obtain continuous culture of cells for at least four weeks;
- to decrease nutrient concentration, thereby reducing running costs;
- to provide a method and apparatus capable of industrial scale-up and application, thereby enabling large scale production of recombinant proteins from plant cell cultures that were hitherto unattainable.

### BRIEF DESCRIPTION OF THE FIGURES

- Figure 1 is a schematic representation of a preferred continuous plant cell bioreactor according to the present invention ;
- Figure 2 is a combined graph of the growth parameters of a continuous cell suspension expressing recombinant human serum albumin carried out with the method of the invention and a bioreactor according to Figure 1 ;
- Figure 3 is a combined graph of the growth parameters of a continuous cell suspension expressing recombinant human lactoferrin carried out with the method of the invention and a bioreactor according to Figure 1 ;
- Figure 4 is a graphical representation of the linear correlation between the packed cell volume (pcv) and the dry weight of cells in suspension for each exemplary protein expressed in the bioreactor;
- Figure 5 is a set of stained preparations of tobacco cell types present in the plant cell suspension in the bioreactor of the present invention;
- Figures 6 and 7 are Western Blots of the expression of recombinant human HSA in the continuous plant cell culture bioreactor of the present invention;
- Figures 8 and 9 are Western Blots of the expression of recombinant human lactoferrin in the continuous plant cell culture bioreactor of the present invention ;
- Figure 10 is a graphical representation of the quantification of expressed recombinant lactoferrin in the bioreactor of the invention over time based on an ELISA analysis.

### DETAILED DESCRIPTION OF THE INVENTION

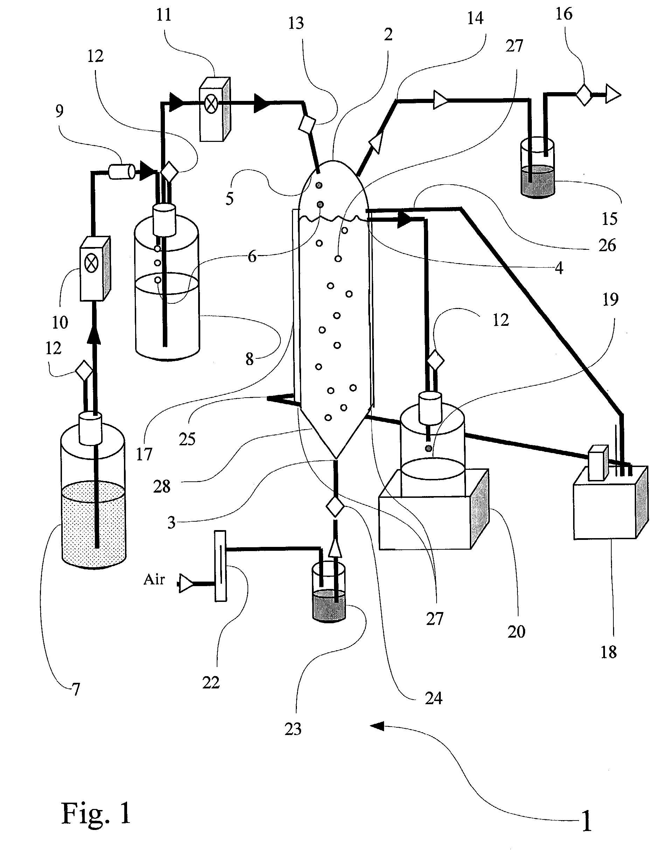

The present invention will be more particularly described by the following detailed description of some preferred embodiments of the invention, in particular in relation to the continuous cell bioreactor illustrated in Figure 1 and production of recombinant human lactoferrin or human serum albumin. It is to be noted that these two proteins are completely different, in that HSA is a non-N-glycosylated protein both natively and in the current recombinant system, whereas lactoferrin is a N-glycosylated protein both natively and when produced in the present recombinant system. Thus the bioreactor of the present invention is capable of producing both N-glycosylated and N-unglycosylated recombinant polypeptides or peptide fragments. This is furthermore one of the advantageous aspects of the current invention.

Figure 1 illustrates a bioreactor according to the invention and capable of continuously producing a desired recombinant therapeutic protein from a plant cell suspension culture.

The bioreactor, generally indicated by the reference numeral 1, comprises a reactor or fermentation chamber 2. The chamber 2 is substantially cylindrical, with an oxygenating fluid inlet 3 located at its bottom, a cell supernatant outlet 4 and a cell media inlet 5 located at its top. The cell media inlet 5 is located substantially opposite the cell supernatant outlet 4, to ensure that any fresh cell media introduced is correctly circulated in the chamber 2 and not drawn off immediately, without having had the chance to grow and differentiate.

The cell media 6 can contain growth media, for example, nutrients. Preferably, the cell media also contains an antioxidant, for example ascorbic acid, although other antioxidants could also be used providing they do not negatively affect the plant cell growth. The cell media is contained in cell media tanks 7 and 8, that are connected in series to cell media inlet 5. Cell media tank 7 contains cell media that is unfiltered. A filter 9 is positioned between an outlet of cell media tank 7 and the entrance to the second media tank 8. The filter 9 can be a 40 micrometer filter, for example a 40 micrometer PALL (UK) Profile II filter, to filter out impurities before the cell media enters the second cell media tank 8. The cell media 6 can be pumped from cell media tank 7 into cell media tank 8 by a peristaltic pump 10. Cell media tank 8 contains filtered cell media 6, which can be pumped via the cell media inlet 5 with another peristaltic pump 11. The outlet of cell media tank 8, as with the other tanks 7 and 19 is equipped with air vent filter 12, for example a 0.22 micrometer filter obtainable from PALL (UK) under the reference AcroVent PTFE. Two further filters 13, for example 0.8 micrometer and 0.22 micrometer, obtainable from PALL (UK) under the reference Supra Membrane Acro 50, are located between the peristaltic pump 11 and the cell media inlet 5. The chamber 2 is thus filled from near the top of the chamber with cell media 6 that is prefiltered in several places before introduction into the chamber 2.

Also near the top of the chamber 2 is an exhaust outlet 14 through which exhaust gases and other material transported by such gases are exhausted from the chamber 2. The exhaust outlet 14 is connected to a washing bath 15 and from there the exhaust gases flow through a 0.22 micrometer filter 16 to the environment outside of the chamber 2. The washing bath 15 also contains a solution of copper sulfate at 1% weight concentration.

The temperature of the chamber 2 is regulated by a temperature regulation system comprising a second chamber 17 that completely surrounds chamber 2. The chamber 17 is dimensioned around the chamber 2 to let a regulating agent flow completely around the latter. In this example, the regulating agent is water that is circulated from a water bath 18 equipped with a pump and that enters the bottom of chamber 17 via conduit 25. The water flows up and around the side of chamber 2 and leaves chamber 17 via the conduit 26 and is returned to the water bath 18. In this way the temperature of the plant cell suspension can be controlled in a regular way.

The chamber 2 also comprises a supernatant outlet 4 located at about three to four millimeters below the upper level of the cell media 6 in the chamber 2. In this way, the cell suspension can be harvested with a majority of extracellular fluid, and a small quantity of cell debris. The cell supernatant exits the chamber 2 via outlet 4 and falls into a harvesting tank 19. An alternative to this is to pass the cell supernatant via a tangential filter that will separate out the cell debris from the extracellular fluid containing the transgenic protein that has been expressed in the chamber 2. The harvesting tank is maintained at a temperature of about 0°Celsius to about 8°Celsius by surrounding it with cooling media, for example, an ice bath or a cooled water bath 20. An alternative arrangement is a water cooled envelope system, whereby an envelope surrounds the harvesting tank 19 and cooled water is pumped through the envelope and around the tank to maintain constant temperature.

As mentioned in a preferred embodiment, the chamber 2 also has sloping lower walls 28 that converge towards to the air inlet 3. This lets the cells in suspension fall down with gravity. The inlet 3 lets air, and optionally carbon dioxide, enter the chamber 2 from its bottom. These gases are introduced via a flow regulator 22 that sets the pressure and flow rate of the gases, which then pass through a washing bath 23, optionally containing a 1% by weight copper sulfate solution, and a 0.22 micrometer filter 24 and finally through inlet 3 into the chamber 2. In this way, the gases enter the chamber and percolate up through the center of the chamber substantially parallel to the side walls of the chamber 2. The gases rise up as bubbles 27 through the cell suspension and in so doing, cause the cells to rise also and be oxygenated. Once the cells reach the top of the chamber 2, the bubbles of gas have been consumed and the rising effect no longer occurs, so the cells fall back down the sides of the chamber 2 and onto the sloping walls 28, thereby falling down back into the stream of rising bubbles 27. The combination of the sloping walls and rising bubbles 27 avoids the need to agitate the suspension with a stirring or mechanical agitating mechanism, and thereby prolongs the life of the cells and enables continuous production and recovery of the protein in the cell suspension. Some detailed examples will now be given of operational procedures used in the production of two recombinant therapeutic proteins.

It should be noted that all of the tubes used in the present apparatus were made from Versilic ™(Saint Gobain), unless otherwise stated. Of course, other suitable materials can be used to meet the same requirements. Similarly, all of the gas connectors used were made of Delrin®, and comprised an O-ring seal and non-return valve, in order to facilitate quick replacement of the various containers while maintaining sterile conditions.

### Example 1

### Plant material:

Transgenic tobacco plants were transformed with recombinant *Agrobacterium tumefaciens* as described by Horsch *et al.* (1985). Plasmid containing the cDNA encoding HSA with a secretion signal peptide was used to transform *N. tabacum* var. PBD6 and plasmid containing the cDNA encoding human lactoferrin was used to transform *N. tabacum* var. xanthi. Homozygote seeds selected after 2 or 3 generations were cultivated *in vitro* on MS basal medium containing 2 % sucrose and 300 mg/L kanamycin. For the establishment of callus cultures, pieces of leaves obtained from *in vitro* grown tobacco plants were cultivated on agar plates (24°C, 16 h photoperiod) of MS medium containing 3 % sucrose, 1 mg/L NAA, 0.2 mg/L kinetin and 200 mg/L kanamycin, pH 5.8. Six weeks later, cell suspensions were initiated by adding 3 to 4 g of friable callus to 50 mL of cell culture medium in sterile 250 mL Erlenmeyer flasks. Cell suspension cultures were grown on MS basal nutrient medium (Sigma M6899, 4.4 g/L) supplemented with 3 % sucrose, 0.5 mg/L folic acid, 1 mg/L 2,4-dichlorophenoxyacetic acid (2,4-D), 0.1 mg/L kinetin, 0.7 g/L 2-[N-Morpholino]ethanesulfonic acid (MES) and 100 mg/L of kanamycin to maintain selection pressure. The medium was adjusted to pH 6.0 (with 1N KOH prior to autoclaving). Cell suspension cultures were grown in darkness at 26°C on an orbital shaker (130 rpm). Cell suspensions were subcultured every 10 days using a 1/5 (v/v) innoculum. After 3 to 4 subcultures, cell growth rate was stabilized. To provide large medium innoculum volumes for bioreactor culture, cell suspension cultures were also maintained in 1 L Erlenmeyer flasks and incubated using the same conditions.

For the culture in the bioreactor, cells were grown in MS basal nutrient medium (Sigma M6899) supplemented with 2 % sucrose, 0.5 mg/L folic acid, 1 mg/L 2,4-D, 0.1 mg/L kinetin, 0.7 g/L MES and 2 mg/L ascorbic acid, 2 mg/L of the antibiotic augmentin to prevent culture contamination and 100 mg/L of kanamycin to maintain selection pressure. pH was adjusted to 6.3 prior to sterilization by filtration (Acro 50, 0.8/0.2 µm, Pall).

For inoculation of the bioreactor, the following was carried out:
150 mL of the ten days old cell suspension were filtered through a 100 µm mesh sieve. The cells were resuspended in 300 mL liquid medium used in the bioreactor culture. All inoculation procedures were carried out using sterile techniques in a laminar flow hood. The culture was maintained at 25.5 ±1 °C and 0.1 vvm air (volumes of gas per volume of media per minute). During the latency phase (2 days), the culture was grown in batch. The culture was transferred directly into the reactor via inlet 5 as identified in Figure 1, that is, the fermentation chamber 2 was disconnected from the general assembly and the inoculating culture introduced via inlet 5, under a laminar flow hood. Such a procedure is adapted to small industrial units since only small amounts of inoculating culture are introduced, at most a few grams. For larger scale industrial production, the inoculating culture can be prepared under a laminar flow hood and the container or recipient containing the inoculating culture can be transferred into the fermentation chamber 2 via the inlet 5, either by gravity, or by using a pump that is designed not to cause any rupture of the cells, when the cells enter the pump body.

After reconnection of the fermentation chamber, fresh medium was injected drop by drop at 150 mL /day flow rate. Under these conditions, the bioreactor was full after 10 to 15 days. The injected air was adjusted to 0.7 vvm.

### Example 2

### Bioreactor design :

The design of the bioreactor is new for plant cell culture (Figure 1). An airlift bioreactor was used to study cell growth and the continuity of the recombinant protein production. The working volume was 2.5 L. The cylinder vessel was made of glass with an external water jacket. A water-bath was used for temperature control by circulating water through the jacket. Aeration was effected through an Acro 50, 0.2 µm sterile inlet air filter (available from Pall, USA) and bubble aeration either by sparger or sintered glass which permitted the agitation of cell suspension. The volume rate was controlled by a Brooks flow meter flow tube R 2 15 AAA, with a stainless steel float (available from Brooks, UK). The exhaust air was evacuated through an Acro 50, 0.2 µm sterile filter (available from Pall, USA). Washing bottles containing a 1% cupric sulfate solution were placed between the bioreactor and the filters at the entry and the exit to humidify the air (Kurz, 1970; Miller *et al.,* 1968). The new medium was injected drop by drop with a peristaltic pump through an Acro 50, 0.8/0.2 µm filter (available from Pall, USA). The tank of medium was sterilized by filtration using a Spiral Cap Supor 0.8/0.2 µm filter (available from Pall, USA) in the laminar flow hood and protected from light. Harvesting of the cells was carried out using an overflow. The harvest tank was maintained at 4°C with ice. The harvest was filtered and deep frozen every day and growth measurements were made.

### Example 3

### Growth measurement:

Dry cell weight: A 100 mL culture sample was centrifuged with a Jouan C 4.11 centrifuge for 5 minutes at 1000 rpm (available from Jouan, France). The pellet was filtered through a 0.45 micrometer Wheaton glass sinter, and washed with distilled water. The water was removed by draining under vacuum until no further drops of water appeared. The biomass was placed on Whatman N°1 filter paper and the fresh weight was measured on a pre-weighed Petri dish. The pellet was dried during 12 h at 80°C and the dry weight was recorded. The percent of dry weight was the ratio (fresh weight/dry weight) x 100.

The packed cell volume (PCV) was determined by centrifuging 5 mL culture suspension in a 15 mL graduated conical centrifuge tube at 1000 rpm for 5 min. The fraction of the whole volume occupied by the cells determined the PCV.

Cell viability: A 20 mL sample of the cell suspension was added with 20 µL of fluorescein diacetate (FDA) according to the method described by Larkin, 1976. The cells were observed using an epifluorescence inverted microscope (Nikon Eclips TE 300) under fluorescent light illumination (filter Nikon B-2E) with a green filter, an excitation wavelength of 450-490 nm, and an emission wavelength 520-538 nm.

Axenic test: For determination of axenic culture, PCA (Plate Count Agar) medium for micro-organisms and YM (Yeast Medium) for yeast and moisture were used. A sample of culture provided by the harvested tank was deposited in the medium and the Petri dish were incubated for 48 h at 28°C.

### Example 4

### Protein extraction:

After filtration on a 100 µm mesh sieve, the harvested culture medium was centrifuged 30 min. at 13000 rpm (available from Sorvall, USA) and stored at -20°C before analysis. For plant cells or leaf tissues, samples were ground directly in extraction buffer (50 mM Tris/HCl, 1 mM EDTA, 100 mM NaCl, pH 7.5), with 0.2% (v/v) Triton X-100 using the ultra-turrax device (available from IKA, Germany) and extracts were clarified by centrifugation at 13,000.g for 10 min. at 4°C. The supernatants were stored at -20°C for further analysis.

Protein concentration was measured according to the Bradford protocol (Bradford, 1976).

### Example 5

### ELISA:

For HSA ELISA the microtiter plates were coated overnight at 4°C with 100 µL of rabbit anti-HSA polyclonal antibody (Sigma) at 1/20000 in 0.05 M carbonate buffer, pH 9.6. Plates were washed with PBS (136.9 mM NaCl; 8.15 mM Na₂HPO₄, 2H₂O; 1.47 mM KH₂PO₄; 2.68 mM KCI; pH 7.4)-Tween 20 [0.05% (v/v)] and then incubated one hour with 300 µL of PBS-tween 20 [0.05% (v/v)] containing 5 % non fat milk. Samples diluted in PBS were then added. Plates were incubated for a further 2 hours at room temperature and washed with PBS-Tween 20 [0.05% (v/v)]. Immunodetection was performed using 100 µL anti-HSA sheep antibody coupled to peroxidase (The Binding Site) diluted at 1/15000 in PBS, incubated 1 hour. Staining was initiated by adding 100 µL of Tetra Methyl Benzidine (TMB) and 50 µL of H₂SO₄ 0.5 M. After a 5 minute incubation, optical density was measured at 450 nm.

For lactoferrin ELISA, the same protocol was used except that monoclonal mouse antibody to human lactoferrin (MAHu/Lfr/Mab, available from Nordic Immunological, Tebu, USA) diluted 1/500 in PBS-Tween was used for the coating. Saturation was performed with 3 % BSA instead of non fat milk and the plates were incubated at 37°C. Anti-lactoferrin antibody conjugated to peroxidase (GAHu/Lfr/PO, available from Nordic Immunological, Tebu, USA) diluted 1/2500 in PBS-Tween was used for immunodetection.

### Example 6

### Western Blot:

The samples from culture medium were analyzed by 10 % or 12 % SDS-PAGE using the LaemmL i buffer system (LaemmL i, 1970). The proteins were transferred to nitrocellulose (Amersham, UK) for immunoblot analysis according to the method described by Towbin *et al.* (1979).

### RESULTS:

### Continuous cell culture parameters

The applicants designed a 2.5 liter airlift bioreactor for continuous plant cell culture. The diameter of the base of the bioreactor was equal to the diameter of the sintered glass through which air was injected and the first lower third of the bioreactor had the form of an inverted cone, with walls sloping towards the air injection inlet. This particular arrangement enabled the applicants to obtain a bioreactor according to the invention that avoided problems of cell sedimentation and also limited shear stress within the chamber.

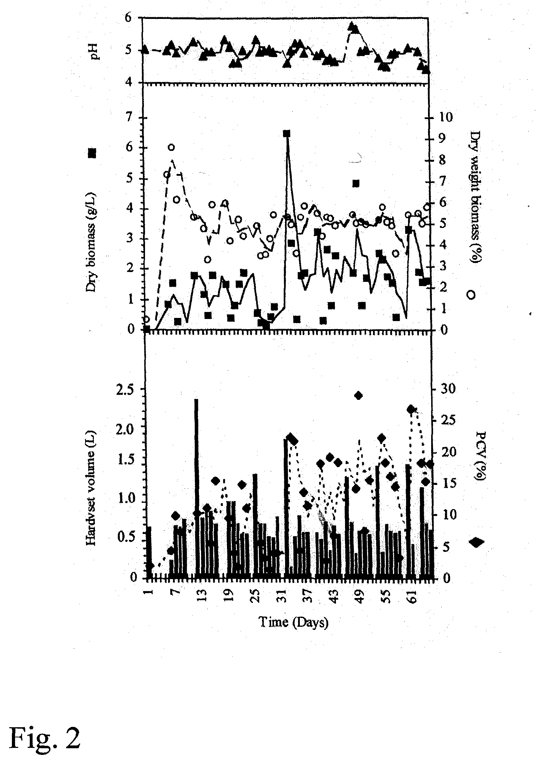

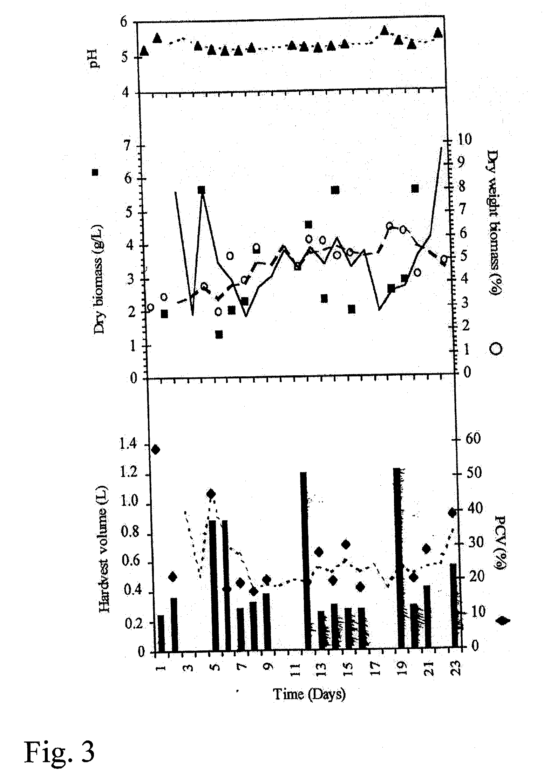

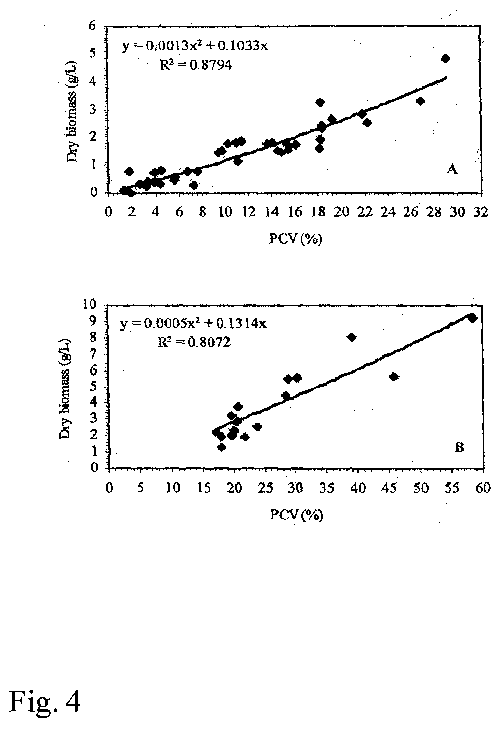

*In vitro* grown transgenic homozygous tobacco plantlets were used to produce calli which permitted the establishment of plant cell suspensions after 6 weeks. Four weeks later, suspension was stabilized and served to initiate the bioreactor. Filling was achieved 10 to 15 days later leading to the start of continuous culture. Two lines of plant cell suspensions, one expressing recombinant human serum albumin (HSA) and the other recombinant human lactoferrin, were used for this study. Preliminary experiments showed a rapid browning of culture and loss of cell viability due to oxidation caused by aeration. The addition of ascorbic acid (2 mg/L) permitted cell culture for between 3 to 10 weeks and viability was stabilized. The applicants also noticed that a healthy suspension culture was characterized by generation of foam at the upper surface of the reactor. To reduce the risk of contamination by microorganisms, sterile filters were used at every entry and exit of the bioreactor and sterile joints with rapid locking were installed at every junction. The bioreactor was inoculated under a laminar flow hood. Axenic tests were regularly performed with PCA medium for micro-organisms and YM for yeast. No contamination was observed during the continuous culture of the HSA and lactoferrin reactors. Growth parameters were followed throughout the duration of the continuous cell culture (Figures 2 and 3) by analysis of the harvested overflowed culture. *N. tabacum* cv PBD6 cells expressing HSA were maintained for 66 days leading to the collection of 34 L of culture medium. *N. tabacum* cv xanthi cells expressing lactoferrin were cultured in the continuous mode for 23 days and 8.2 L of medium was harvested. In both cases, the cell medium was buffered with MES and the pH was relatively stable during cell culture (mean values 5.0 and 5.3 respectively for HSA and lactoferrin cell cultures). Over the period of time used for cell culture, the packed cell volume (pcv) and dry weight fluctuated. This phenomenon can be explained by partial plugging of the outlet valve by cell aggregations that occurs regularly during the overflow of the harvest. However, a linear correlation (Figure 4) between these two parameters could be determined. In any case, the percent of dry weight biomass (Figures 2 and 3) was relatively constant in both cases (about 5% for HSA cells and 5.4% for lactoferrin cells).

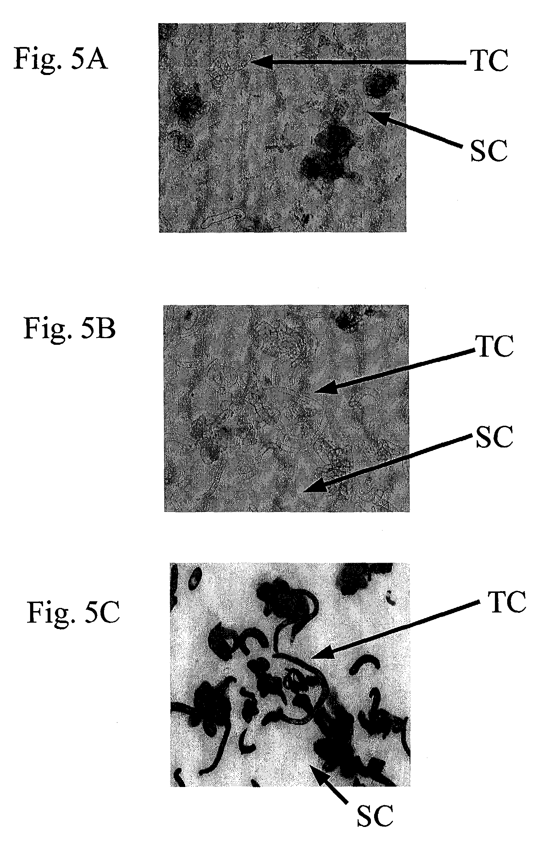

For each cell suspension, microscopic observations were performed (Figure 5). Both suspensions consisted of two cell populations : one type made of spherical (from 20 to 50 µm in diameter) mitotically active cells, and the other one formed by more differentiated cells showing a generally tubular shape (40 to 120 µm length) and often associated as filaments. With no regard for cell type, viability as measured by fluorescein diacetate staining was about 75% all along cell culture.

Parallel to controlling cell growth parameters, bioreactor culture was optimized. With continuous culture, nutrients are not totally consumed so quantities of minerals and energy sources can be reduced without consequence on cell growth. For the HSA producing cells, reduction of sucrose from 3% to 2% was progressively carried out (on days 12, 16, 21 and 33 respectively at 2.7%, 2.5%, 2.2% and 2%) as was a reduction of the MS salts basal medium from 4.4 g/L to 4 g/L (on day 44). No noticeable effect on plant cell growth was observed.

### Production of Recombinant protein

In order to check the efficiency of a plant cell culture system and bioreactor according to the present invention for recombinant protein production, culture medium was analyzed from suspension cultures of cell lines expressing either HSA or lactoferrin genes and compared to expression in plants or calli.

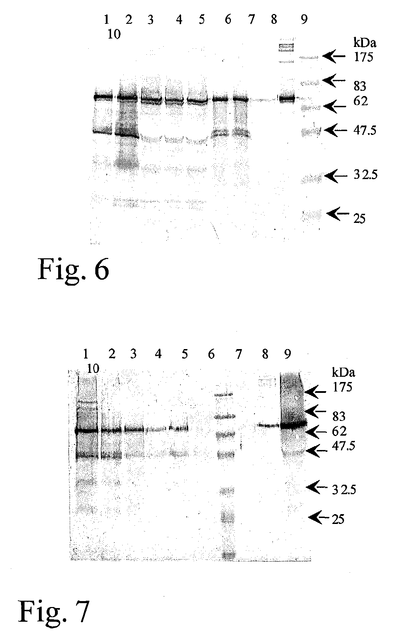

The expression of recombinant HSA in tobacco plants, calli and culture medium can be detected by Western Blot (Figure 6). A specific immunostaining revealed the presence of HSA compared to the control. Two minor bands at 45 kDa in transgenic material probably corresponded to a degradation product from HSA. In a yeast expression system, a 45 kDa band was also observed. This peptide corresponded to an N-terminal fragment of HSA produced by proteolytic cleavage. Others have demonstrated that the 45 kDa fragment in *Saccharomyces cerevisiae* suspension culture was produced by a pH dependent proteolysis mediated by cell-bound protease. As the culture medium in the present invention was buffered, pH was relatively constant and the applicants did not notice a variation in the level of the 45kDa band. Analysis of the culture medium over the period of culture in the continuous bioreactor showed the same pattern of immunostained proteins (Figure 7). However, the intensity for the 66 kDa HSA band, as for the 45 kDa band, decreased with aging of the culture and disappeared after 66 days, which corresponded to death of the cells. The amount of HSA produced over time during continuous culture in the bioreactor was estimated by ELISA. This quantification of HSA was confirmed Western analysis, and it was noted that production decreased after 5 weeks of continuous culture. HSA production during the 30 first days varied from 0.1 to 3.0 µg/mL , with a mean value around 1 µg/mL of suspension medium. In that environment, HSA accounted for about 3% of the total soluble proteins, in contrast to leaves from tobacco plants where HSA represented less than 0.2% of the proteins presents in a leaf extract. In total, 34 liters of culture medium were produced in 66 days leading to a purifiable amount of 23 mg of HSA.

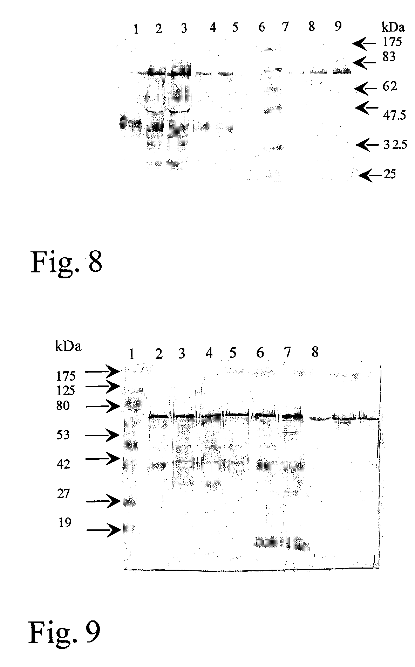

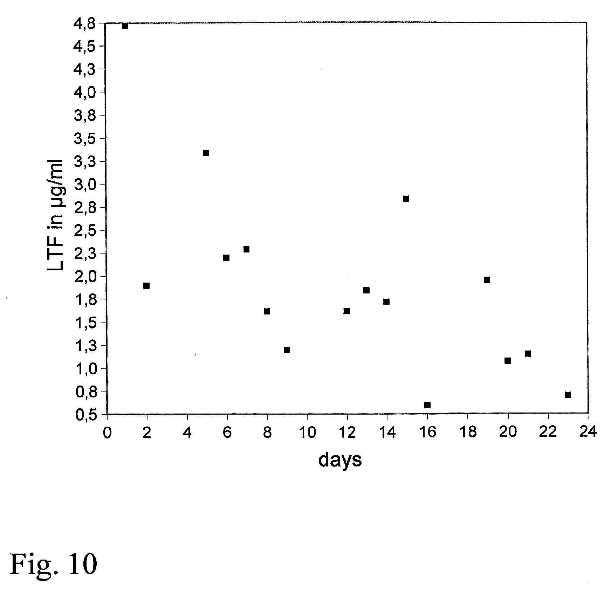

A lactoferrin producing bioreactor was initiated and the production of recombinant protein in cell culture medium was compared to lactoferrin produced in tobacco plants and calli (Figure 8). Western Blot analysis in leaves from *in vitro* plants shows a major immunoreactive band at 80 kDa (lanes 2 and 3) that is absent from non transgenic leaves (lane 6) and corresponds to the recombinant human lactoferrin. Many bands of lower apparent molecular weight are present, with the most important one at about 40 kDa that probably results from proteolytic cleavage of the lactoferrin. In contrast callus extract (lane 1) presents essentially degradation products at about 40 kDa, with a very low level of immunoreactive protein at 80 kDa. However culture of these cells in a continuous suspension mode, enabled the applicants to counterbalance the ratio between these two reactive bands, leading to a major product at 80kDa found in the culture medium (lanes 4 and 5). The applicants were able to pursue continuous culture in the bioreactor of the invention of these cells for 23 days, and lactoferrin production was followed by Western blot (Figure 9). The 80 kDa product does not seem to diminish as the age of cell culture progresses. As an ELISA method takes into account all immunoreactive forms of the peptides, lactoferrin was quantified by measuring the 80 kDa band intensity on a Western Blot and the results represented as a graph (Figure 10). It can be noted that for the first 4 days of continuous culture, part of the lactoferrin measured represents recombinant product accumulated during the filling of the reactor. Then production is relatively stable as culture progresses, varying between 0.75 and 1.3 µg/mL, and giving about 2% of total soluble proteins, whereas the protein expression rate was only 0.1% of total extracted protein in plant leaves. Finally, in the continuous culture (23 days), 8.2 liters of culture medium were collected containing up to 9 mg of recombinant lactoferrin.

### DISCUSSION

In order to test the feasibility of continuous production of recombinant protein from plant cell suspensions, the applicants have designed a bioreactor according to the invention adapted to plant cell culture. It is known that hydrodynamic stress is an important limitation for the plant cell culture in the bioreactor. It is generally admitted that mechanical and hydrodynamic stresses stimulate the plant cell defense mechanism known as an 'oxidative burst'. It is characterized by the production of activated oxygen species (AOS) : superoxide anion, hydroxyl radical and hydrogen peroxide. The AOS produced during the hypersensitive response are toxic to the cells. During previous internal studies carried out by the applicant, after ten days of culture an important browning of the cell suspension concomitant with cell death was observed. To reduce this oxidative stress, ascorbate, which traps free radicals, was added to the culture medium at 2 mg/litre. In this case, and according to one of the preferred embodiments of the invention, it was possible to keep cell suspensions in continuous culture for many weeks with a viability greater than 75 %. Very low cell debris was present. The suspensions were composed basically of two cell populations : one comprising a spherical cell type and the other one comprising tubular cells. Similar populations have been observed in *N. tabacum* cultivated in a continuous mode in a bubble column. This appears not to be due to their culture in the bioreactor of the invention, but rather a characteristic of the applicant's cell suspensions, since the same populations were also observed in Erlenmeyer flasks.

At the air-liquid interface, foam was observed. However, addition of an anti-foaming agent significantly reduced the value of Ka (volumetric mass transfer coefficient) and enhanced cell clumping, adhesion to the bioreactor surface and decreased cell growth. Consequently, the applicants avoided use of such anti-foaming agents in it's system. An alternative to the problem of foam accumulation could be the use of a cell scraper. The layer of foam is composed of proteins and excreted polysaccharides and it increases the viscosity of the culture. However, the applicants did not notice, in the bioreactor according to the invention, a significant increase in viscosity because continuous culture allowed for progressive elimination by overflow. In addition, polysaccharides rich droplets can form a spray and be dispersed in the air in the chamber. In order to avoid closure of the exhaust fluid outlet from such dispersions, it is possible to place a washing bottle containing 1% CuSO₄ upstream of an air filter between the filter and the exhaust exit of the bioreactor.

Fresh medium was introduced into the reactor opposite to the supernatant exit, and this was key in enabling the applicants to keep the composition of the medium in the reactor homogeneous. The minimum dilution rate was set to 0.25 day¹ and average cell concentration was 2 g/L. Without wishing to be limited by theory, the applicants have the following hypothesis to explain the differences in results between the present invention and the known art :
- the strain of tobacco is not the same;
- in the applicants' system, and in the preferred examples, the bioreactor was inoculated with 300 mL (1/8 of total volume) of cell suspension. This suspension was constituted with a cell culture kept in an Erlenmeyer flask in an exponential phase and diluted by half with fresh medium. After a lag phase (2 or 3 days), the bioreactor was constantly and slowly filled with fresh medium in order to keep cells in an active growth stage;
- the medium composition is different in the preferred embodiments, particularly concerning the concentration of KH₂PO₄ ;
- the aeration rate used was about 0.7 vvm for the applicant's system, leading to a different volumetric oxygen transfer coefficient (K_{I}a), since this value is dependent on many parameters such as the aeration system, aeration rate of the bioreactor and composition of the nutrient solution. In the applicant's bioreactor according to the present invention, the aeration rate was selected in order to avoid cell sedimentation.

As has been demonstrated by the results shown here in the present application, production of recombinant proteins in cell suspensions have advantages over stable or transient plant production when milligrams of proteins need to be rapidly produced. Actually, a lower amount of total soluble proteins is present in a culture medium compared to leaf extract, and the purification process of the secreted recombinant protein is thereby easier to carry out. Comparison of recombinant HSA and recombinant lactoferrin levels produced in the bioreactor and tobacco leaves showed a mean 15 to 20 fold enrichment in recombinant protein secreted in the culture medium. Expression levels that were obtained for HSA (mean 1 µg/mL) and lactoferrin (0.75 to 1.3 µg/mL) in the bioreactor were higher than those obtained by others for a recombinant growth factor (0.25 µg/mL), for an interleukin-4 (0.18 µg/mL) and for a scFv (0.5 µg/mL) antibody fragment produced in tobacco cell suspension culture.

In order to work in optimized conditions, the applicants used cell suspension cultures obtained from stable transgenic homozygous tobacco plants. Indeed, the cultures were considered as clones and no variability in protein expression could be attributed to differential gene expression. However, it is also perfectly feasible to start from tobacco leaf or explants that have been transformed by the routine plant transformation techniques to obtain transgenic callus and from there, transgenic cell suspensions expressing the desired recombinant protein.

Cell renewal during continuous culture enabled maintenance of the cells in a correct stage of their life cycle for recombinant protein production. Indeed, the harvested culture medium was analyzed each day and found to continuously secrete proteins until the cell died. The applicants were able to run the bioreactors for 66 days and 23 days respectively for HSA and lactoferrin producing cells and to harvest 34 liters and 8.2 liters of culture medium. The total amount of recombinant proteins produced was about 23 mg for HSA and 9 mg for lactoferrin. Automation of the measurement of many parameters under computer control, for example, oxygen rate, flow rate, and adjustments during culture allowed for improvement of the stabilization of the physiological stage of the cells, thereby enabling the continuous culture to run as long as the protein to be expressed was needed. The applicant's system and method according to the invention has proven to be really interesting compared to batch culture, since the protein is continuously produced and bioreactor immobilization is limited.

The pattern of immunoreactive peptides revealed with anti-HSA antibodies is similar between plant leaves, calli and culture medium. The 66 kDa band observed was a secreted HSA with its signal peptide deleted (data confirmed by N-terminal sequencing) which is consistent with previous demonstrations of correct cleavage of the same HSA precursor in potato and tobacco cells. Some degradation products were also detected.

The pattern of the immunostained bands for the lactoferrin producing bioreactor was comparable to the ones produced in tobacco leaves. Thus, the 80 kDa band observed in the cell suspension medium corresponded to lactoferrin, the signal peptide of which had been cleaved. The 80 kDa band was the major immunostained product in the culture medium, but this is not the case for plant calli. The lower bands come from an extracellular proteolytic processing of the lactoferrin, and that dilution caused by the culture medium limits contact between proteases in the extracellular matrix and the expressed lactoferrin. Similar results have been found with respect to proteolytic fragments of a As32-scFv protein in culture medium in contrast to crude extracellular protein fractions from stable transgenic tobacco plants. However, when the cell culture aged, a low MW immunoreactive band was observed indicating an additional degradation of the protein. This protein cleavage was probably linked to cell death and release of proteolytic enzymes in the culture medium.

The applicants have thus successfully produced a new airlift bioreactor functioning with a conical bubble column for continuous cell suspension culture, and have demonstrated that continuous culture was possible for several weeks. Furthermore, the applicants have also demonstrated that transgenic plant cells could keep producing large amounts of recombinant protein in the culture medium. Applicants have also effected purification of the recombinant proteins expressed from the culture medium and further characterization of the proteins has been carried out. The proteins have been analyzed for their amino acid sequences and any post-translational modifications. Fine structural comparison of proteins produced in plants and cell suspension was also carried out. The results obtained support in particular the real efficiency of tobacco cell suspension cultivated in a bioreactor according to the present invention for producing mutliple milligram quantities of recombinant therapeutic proteins in only a few weeks.

Purification of rHSA from the tobacco cell bubble lift bioreactor according to the invention.

A two step protocol was used to purify HSA secreted in the bioreactor, using hydrophobic interaction chromatography (HIC) followed by anion exchange chromatography (AEX). This purification process forms yet another aspect of the present invention.

### Hydrophobic Interaction Chromatography

Culture filtrate was previously prepared by filtration through a 100 µm mesh and centrifugation at 8000 rpm for 30 minutes, at 4°C. The resultant supernatant was stored at -20°C. Culture filtrate that had been filtered through 0.45 µm filter showed that the HSA was intact.

Culture filtrate was defrosted quickly in a waterbath at 30°C, and adjusted to a final concentration of 50 mM with 1 M Tris-HCl buffer, pH 8.0. To this was added ammonium sulphate (AMS) to produce a 37 % saturated solution (222 gL⁻¹). The solution was filtered through Mira cloth and loaded onto a 5 mL Butyl-Toyopearl 650 M column equilibrated with 50 mM Tris-HCl buffer, pH 7.5 containing 37 % saturated AMS. The elution gradient, 0-60 % Buffer B (Tris buffer without AMS) was designed to remove endogenous proteins. A step to 100 % B elutes recombinant HSA. The pooled fractions were dialysed against 20 mM Tris-HCl, pH 8.0

### Anionic Exchange Chromatography

The dialysed sample was loaded onto a 4 mL column of Neobar AQ, equilibrated with 25 mM Tris-HCl, pH 8.0. A gradient of 50-200 mM NaCl in this buffer removed remaining endogenous proteins. Recombinant HSA was then eluted with a 400 mM NaCl in 25 mM Tris-HCI buffer, pH 8.0, in only a few fractions. The pooled elution fractions (2-4 mL in volume) were stored at -70°C.

### Electrophoresis

The samples were precipitated directly with 80% acetone at -20 °C overnight, resolubilised in denaturing SDS-PAGE buffer and analysed on 9 % acrylamide gels. For N-terminal sequencing, lanes were loaded with 200-400 ng (3-6 pmoles) of recombinant HSA, and following electrophoresis and western blotting onto PVDF membranes, the excised bands were sent for analysis. The correct HSA N-terminal sequence of DAHKS was obtained. This demonstrated correct signal peptide sequence cleavage.

### Results

It was therefore shown that recombinant HSA could be purified from the bioreactor according to the present invention.

Electrophoretic analysis of recombinant HSA containing unpurified culture filtrate suggested the presence of a contaminating endogenous protein with an almost identical mobility to HSA. During HIC the majority (estimate 90%) of this particular contaminant was removed in the gradient elution. During subsequent AEX it eluted in the wash step, prior to recombinant HSA. Thus both steps can be used together, to remove it, from the recombinant HSA preparation. Interestingly, it was found that precipitation with acetone (prior to SDS-PAGE) was vastly superior to that with TCA. In addition, cation exchange chromatography (CEX) at pH 4.5 (which can be substituted for AEX) resulted in an apparent increase in degradation bands. These observations may indicate an acidic- induced degradation of HSA, similar to that observed for elution from immunoaffinity matrices.

The two step protocol purified recombinant HSA to a "coomassie blue purity level" (i.e., only 1 band observed) and subsequent silver staining showed a few minor other bands. Immunostaining showed principally intact HSA (approx. 66 kDa); and only minor clipped products. The yield from 80 mL of culture filtrate was about 5 µg of pure HSA. Both chromatography steps are relatively simple to scale-up. The recombinant HSA co- chromatographed with plasma HSA on both HIC and AEX. In addition, the N-terminal amino acid sequence was identical to that of plasma HSA. These data confirm that this process will yield essentially pure HSA from a tobacco cell bioreactor culture filtrate feedstock. It was noted that the recombinant HSA obtained from the cell culture was substantially purer than the commercially available non-recombinant product obtained from plasma used for comparison, in that the recombinant HSA showed a reduced tendency to aggregation, which is indicative of a lower amount of impurities such as dimers and trimers of the molecule.

### Bibliography :

Kato A, Kawazoe S, lijima M, Shimizu Y : J. Ferment. Technol., 54, pages 82-87, 1976, "Continuous culture of tobacco cells."

Horsch RB, Fry JE, Hoffman NL, Eichholtz D, Rogers SG, Fraley RT : Science, 227, pages 1229-1231, 1985, "A simple and general method for transferring genes into plants."

Miller RA, Shyluk JP, Gamborg OL, Kirkpatrick JW : Science, 159, pages 540-542, 1968, "Phytostat for continuous culture and automatic sampling of plant cell suspensions.

Kurz WGW : Explt. Cell Res. 64, pages 476-479, 1970 : "A chemostat for growing higher plants in single cell suspension cultures."

Bradford MM : Anal. Biochem. 72, pages 248-254, 1976 : "A rapid and sensitive method for the quantitation of microgram quantities of protein utilizing the principle of protein dye binding."

Laemmli UK : Nature, 227, pages 680-685, 1970 : "Cleavage of structural proteins during the assembly of the head of the bacteriophage T4."

Towbin H, Staehlin T, Gordon J : Proc. Natl. Acad. Sci, 76, pages 4350-4354, 1979 : "Electrophoretic transfer of proteins from polyacrylamide gels to nitrocellulose sheets : Procedure and some application."

Larkin PJ : Planta, 128, pages 213-216, 1976 : "Purification and viability determination of plant protoplasts."

## Claims

1. Continuous plant cell bioreactor comprising :
- a fermentation chamber;
- an oxygenating inlet enabling oxygenating fluid to enter the chamber;
- a cell media inlet enabling cell media to enter the chamber;
- and a plant cell supernatant outlet enabling cell supernatant to exit the chamber;
wherein the cell media inlet and the supernatant outlet are located substantially one opposite the other.

2. Continuous plant cell bioreactor according to claim 1, wherein the reactor is a stirred tank bioreactor or an airlift bioreactor, and preferably is an airlift bioreactor.

3. Continuous plant cell bioreactor according to any preceding claim, wherein the bioreactor is an airlift bioreactor chosen from the group consisting of an external-loop airlift reactor, an internal-loop airlift reactor, and a bubble column reactor.

4. Continuous plant cell bioreactor according to any preceding claim, wherein the reactor is a bubble column airlift bioreactor.

5. Continuous plant cell bioreactor according to any preceding claim, wherein the fermentation chamber is substantially elongate in shape, preferably substantially rectangularly cubic, and even more preferably, substantially cylindrical.

6. Continuous plant cell bioreactor according to any preceding claim, wherein the bottom of the fermentation chamber has substantially sloping walls that converge towards the oxygenating inlet.

7. Continuous plant cell bioreactor according to any preceding claim, wherein the bioreactor further comprises an inoculating suspension inlet for introducing an inoculating cell suspension.

8. Continuous plant cell bioreactor according to any preceding claim, wherein the cell media inlet and the inoculating suspension inlet are one and the same.

9. Continuous plant cell bioreactor according to any preceding claim, wherein the cell media inlet, the inoculating suspension inlet and the plant cell supernatant outlet are located substantially at the top of the chamber.

10. Continuous plant cell bioreactor according to any preceding claim, wherein the oxygenating inlet is located substantially at the bottom of the chamber.

11. Continuous plant cell bioreactor according to any preceding claim, wherein the operational reactor temperature is comprised between about 23°C and about 27°C.

12. Continuous plant cell bioreactor according to any preceding claim, wherein the oxygenating inlet comprises a filter.

13. Continuous plant cell bioreactor according to any preceding claim, wherein the oxygenating inlet comprises a sintered glass filter.

14. Continuous plant cell bioreactor according to any preceding claim, wherein the oxygenating inlet comprises a filter having a pore diameter comprised between about 17 micrometers and about 100 micrometers.

15. Continuous plant cell bioreactor according to any preceding claim, wherein the oxygenating inlet comprises a sintered glass filter.

16. Continuous plant cell bioreactor according to any preceding claim, wherein the oxygenating fluid is air.

17. Continuous plant cell bioreactor according to any preceding claim, wherein the oxygenating fluid is oxygen.

18. Continuous plant cell bioreactor according to any preceding claim, wherein the oxygenating fluid in the chamber is a gas of density lower than that of a cell culture within the chamber.

19. Continuous plant cell bioreactor according to any preceding claim, wherein the cell supernatant outlet is located below an upper level of a cell culture in the chamber.

20. Continuous plant cell bioreactor according to any preceding claim, wherein the bioreactor further comprises a chamber temperature regulation system.

21. Continuous plant cell bioreactor according to any preceding claim, wherein the bioreactor further comprises a water-cooled chamber temperature regulator system disposed around an exterior peripheral wall of the chamber.

22. Continuous plant cell bioreactor according to any preceding claim, wherein the bioreactor further comprises a water- cooled chamber temperature regulator system in which cooled water enters the regulator system near the bottom of the chamber.

23. Continuous plant cell bioreactor according to any preceding claim, wherein the bioreactor further comprises a water- cooled chamber temperature regulator system in which cooled water enters the regulator system near the top of the chamber.

24. Continuous plant cell bioreactor according to any preceding claim, wherein the bioreactor further comprises a water-cooled regulator system that is coiled around an exterior peripheral wall of the chamber.

25. Continuous plant cell bioreactor according to any preceding claim, wherein the bioreactor further comprises a water-cooled regulator system that is in direct or indirect contact with the exterior peripheral wall of the chamber.

26. Continuous plant cell bioreactor according to any preceding claim, further comprising at least one separate cell media tank connected to the cell media inlet.

27. Continuous plant cell bioreactor according to any preceding claim, wherein the bioreactor comprises two cell media tanks disposed in series, between which at least one filter is located.

28. Continuous plant cell bioreactor according to any preceding claim, wherein an oxygenating fluid source under pressure is also provided.

29. Continuous plant cell bioreactor according to any preceding claim, wherein at least one filter is provided between the oxygenating fluid source and the oxygenating fluid inlet.

30. Continuous plant cell bioreactor according to any preceding claim, wherein the reactor also comprises at least one harvesting tank, connected to the plant cell supernatant outlet.

31. Continuous plant cell bioreactor according to any preceding claim, wherein the reactor also comprises an exhaust fluid outlet for evacuating exhaust fluids from the chamber.

32. Continuous plant cell bioreactor according to any preceding claim, wherein the operational oxygenating fluid flow rate is comprised from about 0.1 vvm to about 1 vvm, and preferably from about 0.1 vvm to about 0.7 vvm.

33. Continuous plant cell bioreactor according to any preceding claim, wherein the cell media contained in the cell tanks is a suspension of embryonic undifferentiated plant cells.

34. Continuous plant cell bioreactor according to any preceding claim, wherein the cell media contained in the cell tanks is a suspension of transgenic plant cells expressing a recombinant polypeptide or peptide fragment, preferably selected from N-glycosylated and N-unglycosylated polypeptides or peptide fragments.

35. Continuous plant cell bioreactor according to claim 34, wherein the recombinant polypeptide or peptide fragment expressed by the cell suspension is a therapeutic protein.

36. Continuous plant cell bioreactor according to claim 35, wherein the therapeutic protein is selected from the group consisting of antibodies, antigens, antibody fragments, antigen fragments, cardiovascular proteins, digestive enzymes, cerebro-stimulatory or modulatory enzymes, pulmonary enzymes, anti-tumour proteins, tissular proteins, immunostimulatory or modulatory proteins, and blood or blood derived proteins, bone morphogenesis proteins, and growth hormones.

37. Continuous plant cell bioreactor according to any preceding claim, wherein the cell media also contains growth media comprising basic nutrients required for cell viability.

38. Continuous plant cell bioreactor according to any preceding claim, wherein the cell media also comprises an antioxidant in an amount comprised between about 2 to about 4 mg/Litre.

39. Continuous plant cell bioreactor according to any preceding claim, wherein the bioreactor also comprises a control unit for regulating operation of the reactor.

40. Continuous plant cell bioreactor according to any preceding claim, wherein the reactor also comprises a tangential filtration unit located between the cell supernatant outlet and the harvest tank.

41. Method for the continuous culture of a plant cell suspension, comprising :
- introducing a plant cell suspension into a fermentation chamber;
- introducing an oxygenating fluid into the fermentation chamber
whereby the oxygenating fluid is introduced in such a way as to define a circulatory path sufficient to maintain the viability of the plant cell suspension.

42. Method according to claim 41, wherein the chamber is substantially as claimed in any one of claims 1 to 40.

43. Method according to any one of claims 41 or 42, wherein the oxygenating fluid is introduced at the bottom of the fermentation chamber.

44. Method according to any one of preceding claims 41 to 43, wherein the fermentation chamber is maintained at an operating temperature comprised between about 23 to about 27°C.

45. Method according to any one of preceding claims 41 to 44, wherein the oxygenating fluid is air.

46. Method according to any one of preceding claims 41 to 45, wherein the oxygenating fluid is introduced at a flow rate comprised between about 0.1 vvm to about 1 vvm, and preferably from about 0.1 vvm to about 0.7 vvm.

47. Method according to any one of preceding claims 41 to 46, wherein the cell media contained in the cell tanks is a suspension of embryonic undifferentiated plant cells.

48. Method according to any one of preceding claims 41 to 47, wherein the cell media contained in the cell tanks is a suspension of transgenic plant cells expressing a recombinant polypeptide or peptide fragment.

49. Method according to any one of preceding claims 41 to 48, wherein the recombinant polypeptide or peptide fragment expressed by the cell suspension is a therapeutic protein.

50. Method according to any one of preceding claims 41 to 49, wherein the therapeutic protein is selected from the group consisting of antibodies, antigens, antibody fragments, antigen fragments, cardiovascular proteins, digestive enzymes, cerebro-stimulatory or modulatory enzymes, pulmonary enzymes, anti-tumour proteins, tissular proteins, immunostimulatory or modulatory proteins, and blood or blood derived proteins, bone morphogenesis proteins, and growth hormones.

51. Method according to any one of preceding claims 41 to 50, wherein the cell supernatant containing an expressed protein of interest is separated in a tangential filter to separate out cell debris and recover the supernatant.

52. Method according to any one of preceding claims 41 to 51, wherein the oxygenating fluid is introduced in such a way as to produce bubbles having an average diameter at the point of introduction comprised between about 17 micrometers and 100 micrometers.

53. Method according to any one of preceding claims 41 to 52, wherein the oxygenating fluid is passed through a sintered glass filter just prior to introduction into the chamber via the oxygenating fluid inlet.

54. Method according to any one of preceding claims 41 to 53, wherein the cells in suspension are made to fall onto the lower walls of the chamber that slope towards the oxygenating inlet.

55. Method according to any one of preceding claims 41 to 54, wherein the exhaust fluids produced in the chamber are exhausted via an exhaust outlet naturally.

56. Method according to any one of preceding claims 41 to 55, wherein the cell supernatant is collected by overflow of the cell suspension in the chamber into the cell supernatant outlet.

57. Method according to any one of preceding claims 41 to 56, wherein the cell media is introduced into the chamber via the cell media inlet substantially opposite to the cell supernatant outlet.

58. Method according to any one of preceding claims 41 to 57, wherein the plant cell suspension contained in the cell media has a dry cell weight comprised between about 0.2 to about 6 g/Litre.

59. Method according to any one of preceding claims 41 to 58, wherein the oxygenating fluid is additionally periodically introduced in bursts at a greater flow rate than the operational flow rate.

60. Method for the purification of recombinant HSA secreted from a cell culture suspension, comprising the steps of :
- effecting hydrophobic interaction chromatography (HIC) on the recombinant cell culture suspension;
- effecting anion exchange chromatography (AEX) on an eluate obtained from the HIC step.

61. Method according to claim 60, wherein the cell culture suspension is filtered, and the filtrate undergoes HIC.

62. Method according to claim 60 or 61, wherein the eluate obtained from the HIC step is dialysed before undergoing AEX.
